(19) Europäisches Patentamt
European Patent Office
Office européen des brevets

(11) **EP 4 393 961 A1**

(12) **EUROPEAN PATENT APPLICATION**
published in accordance with Art. 153(4) EPC

(43) Date of publication:
**03.07.2024 Bulletin 2024/27**

(21) Application number: **22882742.4**

(22) Date of filing: **13.10.2022**

(51) International Patent Classification (IPC):
**C08B 31/04** (2006.01)  **C08B 33/02** (2006.01)
**A23L 33/125** (2016.01)  **A61P 29/00** (2006.01)

(52) Cooperative Patent Classification (CPC):
**Y02P 20/54**

(86) International application number:
**PCT/CN2022/125134**

(87) International publication number:
**WO 2023/066132 (27.04.2023 Gazette 2023/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.10.2021 CN 202111213344**

(71) Applicant: **Shandong Shanwei Immunotech Co.,
Ltd.
Jinan, Shandong 250014 (CN)**

(72) Inventors:
• **MACKAY, Charles Reay**
**Jinan, Shandong 250014 (CN)**
• **SONG, Yingying**
**Jinan, Shandong 250014 (CN)**
• **QU, Xinyan**
**Jinan, Shandong 250014 (CN)**
• **WANG, Quanbo**
**Jinan, Shandong 250014 (CN)**

(74) Representative: **Huang, Liwei
Cäcilienstraße 12
40597 Düsseldorf (DE)**

(54) **STARCH-INDOLE ACID DERIVATIVE, PREPARATION METHOD THEREFOR, AND USE THEREOF**

(57) The present application provides a starch-indole-acid derivative, a preparation method therefor, and a use thereof and relates to the technical field of modified starch. The starch-indole-acid derivative refers to esterified starch generated by starch undergoing an esterification reaction together with indole acid under the action of a condensing agent and a base. The derivative has high resistance and can resist degradation in the stomach and small intestine. After reaching the colon, the derivative can be fermented by intestinal flora to release indole acid, which is beneficial to intestinal health. Compared with traditional administration methods such as gavage and intraperitoneal injection, the described means has significant advantages and can significantly increase the content of indole acid in colon and hepatic portal blood. In addition, indole acid can activate aryl hydrocarbon receptors to play an immunomodulatory role, which can be combined with the regulatory effect of starch releasing short-chain fatty acids by undergoing fermentation by intestinal flora, providing a product that synergistically exerts immune regulatory effects by means of multiple immune system signaling pathways.

FIG. 1

**Description**

**Cross Reference to the Related Applications**

This application claims the priority of Chinese patent application No. 202111213344.7, filed to CNIPA on October 19, 2021, and entitled "Starch-Indole-Acid Derivative, Preparation Method Therefor, and Use Thereof", the entire contents of which are incorporated herein by reference.

**Technical Field**

The present application relates to the technical field of modified starch, in particular to a starch-indole-acid derivative, a preparation method therefor, and use thereof.

**Background**

Indole acid derivatives such as indole-3-acetic acid (IAA), indole-3-propionic acid (IPA), indole-3-lactic acid (ILA) and indole-3-acrylic acid (IA) are intestinal microbial metabolites of tryptophan and play an important role in regulating intestinal immune balance and the like. Research has found that IAA, IA, ILA, etc. can act as ligands for aryl hydrocarbon receptors (AhRs) to regulate intestinal immune balance, and IAA inhibits the level of pro-inflammatory cytokines in mouse macrophages and hepatocytes in an AhR-dependent manner, thereby relieving liver inflammatory response. IPA regulates the intestinal barrier function in mice by acting as a ligand for a pregnane X receptor (PXR), especially in the presence of indole, and the increase of the concentration of IPA in serum is related to the reduction of morbidity of type 2 diabetes, insulin secretion, and insulin sensitivity; and in addition, IPA may also play an antioxidant effect as a hydroxyl radical scavenger.

Tryptophan is an essential amino acid in humans, and provided by dietary proteins. There are three main pathways for tryptophan metabolism in a gastrointestinal tract: a kynurenine pathway, a 5-hydroxytryptamine pathway, and direct decomposition through intestinal microorganisms. The intestinal microorganisms are the most abundant in a colon, which is a main site for decomposing tryptophan to produce indole acids such as IAA/IPA. Research has found that the content of IAA in feces of obese and diabetic patients is significantly reduced compared with normal populations, and epidemiological studies have shown that the level of IPA in serum is negatively correlated with type 2 diabetes (T2D) and low-grade inflammation, indicating that the content of indole acids such as IAA/IPA is closely related to human diseases. Therefore, targeted colonic delivery of indole acids such as IAA/IPA is expected to play a role in preventing or treating inflammatory diseases and autoimmune diseases such as inflammatory bowel disease, type 1 diabetes, systemic lupus erythematosus, rheumatoid arthritis, autoimmune liver disease and multiple sclerosis by means of the good immunomodulatory efficacy of indole acids such as IAA/IPA.

However, if such indole acid small molecules are ingested directly by oral administration, they will be absorbed in the stomach or small intestine of a digestive tract and cannot effectively reach a colonic site. How to achieve targeted delivery of these indole acid small molecules with immunomodulatory effects to the colonic site and exert their effects is a problem to be solved.

In view of this, the present application is specifically proposed.

**Summary**

A first object of the present application is to provide a starch-indole-acid derivative. The starch-indole-acid derivative can resist degradation in the stomach and small intestine, and after reaching a colonic site, the starch-indole-acid derivative can be fermented by intestinal flora to release indole acid. Compared with traditional administration methods such as gavage and intraperitoneal injection of indole acid, the described means has significant advantages and can significantly increase the content of indole acid in colon and hepatic portal blood.

A second object of the present application is to provide a preparation method for the starch-indole-acid derivative.

A third object of the present application is to provide use of the starch-indole-acid derivative, wherein the starch-indole-acid derivative can be widely used in the preparation of a product for regulating intestinal immune balance.

In order to achieve the above objects of the present application, the following technical solutions are specifically adopted:

the present application provides a starch-indole-acid derivative, wherein the starch-indole-acid derivative is prepared by an esterification reaction between starch and indole acid under the action of a condensing agent and a base; and preferably, the starch-indole-acid derivative after condensation has an indole acid substitution degree of 0.01-1.0.

Further, the indole acid includes at least one selected from indoleacetic acid, indolepropionic acid, indoleacrylic acid or indolelactic acid.

Further, the starch includes at least one selected from high-amylose maize starch (HAMS), potato starch, sweet potato starch, mixed bean starch and banana starch.

Further, the condensing agent includes at least one selected from EDCI, DCC and HATU; and preferably, the base includes at least one selected from 1-methylimidazole, amines, sodium bicarbonate, and sodium carbonate.

The present application provides a preparation method for the starch-indole-acid derivative, including the steps of:

(a) dissolving starch in a solvent to obtain a solution A; and subsequently adding indole acid, a condensing agent and a base into the solution A, and carrying out an esterification reaction to obtain a reaction solution A; and
(b) precipitating the starch in the reaction solution A, followed by successively performing suction filtration and drying to obtain the starch-indole-acid derivative.

Further, the solvent in the step (a) includes at least one selected from DMSO, ionic liquid, and water.

Further, the esterification reaction in the step (a) is carried out at a temperature of 20-80°C for 20-30 h.

Further, the precipitating in the step (b) is carried out by adding dropwise the reaction solution A into ethanol or water to precipitate the starch-indole-acid derivative.

The present application provides use of the starch-indole-acid derivative in the preparation of a product for regulating intestinal immune balance.

Regulating the intestinal immune balance means that after being ingested and reaching a colonic site, the starch-indole-acid derivative is fermented by intestinal flora to release linked indole acid, which cooperates with short-chain fatty acids released by fermentation of starch by intestinal flora, synergistically exerting immunomodulatory effects by means of multiple immune system signaling pathways.

Further, the product for regulating the intestinal immune balance includes a drug, a health food, a formula food for special medical use or a common food.

Further, the drug is a drug for the prevention and treatment of inflammatory diseases and/or autoimmune diseases.

Further, the inflammatory diseases and/or the autoimmune diseases include one selected from inflammatory bowel disease, type 1 diabetes, systemic lupus erythematosus, rheumatoid arthritis, autoimmune liver disease, and multiple sclerosis.

Compared with the prior art, the beneficial effects of the present application are as follows: according to the starch-indole-acid derivative provided by the present application, the starch-indole-acid derivative is mainly prepared by the esterification reaction between the starch and the indole acid under the action of the condensing agent and the base; the starch-indole-acid derivative is acylated starch formed by the esterification reaction between the starch and the indole acid, has high resistance and can resist degradation in the stomach and small intestine. After reaching the colonic site, the starch-indole-acid derivative can be fermented by intestinal flora to release indole acid. Compared with traditional administration methods such as gavage and intraperitoneal injection of indole acid, the described means has significant advantages and can significantly increase the content of indole acid in colon and hepatic portal blood.

The indole acid released by the starch-indole-acid derivative provided by the present application at the colonic site exerts immunomodulatory effects by activating AhR. At the same time, the short-chain fatty acids produced by fermentation of the starch by the intestinal flora may also play an immunomodulatory role by activating G protein-coupled receptors and inhibiting histone deacetylases. The effects of the indole acid and the short-chain fatty acids are positively combined, which can provide a product that synergistically exerts immunomodulatory effects by means of multiple immune system signaling pathways.

According to the preparation method for the starch-indole-acid derivative provided by the present application, the preparation method includes first dissolving the starch in the solvent to obtain the solution A; subsequently adding the indole acid, the condensing agent and the base into the solution A, and carrying out the esterification reaction to obtain the reaction solution A; and then precipitating the starch in the reaction solution A, followed by successively performing suction filtration and drying to obtain the starch-indole-acid derivative. The above preparation method has the advantages of simple processing technology and suitability for industrial large-scale production.

The above starch-indole-acid derivative provided by the present application can be widely used in the product for regulating the intestinal immune balance, and a product for preventing and treating inflammatory diseases.

## Brief Description of the Drawings

In order to illustrate the specific embodiments of the present application or the technical solutions in the prior art more clearly, the drawings required to be used in the description of the specific embodiments or the prior art will be briefly described below. Obviously, the drawings in the following description are some embodiments of the present application, and those of ordinary skill in the art can obtain other drawings based on these drawings without inventive steps.

FIG. 1 is nuclear magnetic resonance hydrogen spectrum of a starch-indole-acid derivative provided in Example 1 of the present application;

FIG. 2 is an infrared spectrum of starch (HAMS) and indoleacetic acid derivatized high-amylose maize starch (HAMSIAA) provided in Example 1 of the present application;

FIG. 3 is an X-ray diffraction (XRD) pattern of starch (HAMS) and indoleacetic acid derivatized high-amylose maize starch (HAMSIAA) provided in Example 1 of the present application;

FIG. 4 is a scanning electron microscope (SEM) image of starch (HAMS) and indoleacetic acid derivatized high-amylose maize starch (HAMSIAA) provided in Example 1 of the present application;

FIG. 5 is a graph showing the concentration of indole acid in feces of experimental mice provided in Example 2 of the present application;

FIG. 6 is a graph showing the concentration of indole acid in hepatic portal blood of experimental mice provided in Example 2 of the present application;

FIG. 7 is a graph showing the concentration of indole acid in feces of experimental mice provided in Example 3 of the present application;

FIG. 8 shows the concentration of IAA in colonic contents for different modes of administration provided in Example 4 of the present application;

FIG. 9 shows the concentration of IAA in hepatic portal blood serum for different modes of administration provided in Example 4 of the present application;

FIG. 10 is a graph showing the concentration of IPA in feces of experimental mice provided in Example 5 of the present application;

FIG. 11 is a comparative diagram of IAA/IPA concentrations in peripheral blood serum in a normal group and a dextran sulfate sodium (DSS)-induced ulcerative colitis group provided in Example 6 of the present application;

FIG. 12 is a graph showing changes in a body weight of mice after 15% HAMSIAA-0.49 treatment provided in Example 6 of the present application;

FIG. 13 is a graph showing changes in a colon length of mice after 15% HAMSIAA-0.49 treatment provided in Example 6 of the present application;

FIG. 14 is a graph showing changes in a disease active index (DAI) of mice after 15% HAMSIAA-0.49 treatment provided in Example 6 of the present application;

FIG. 15 is a graph showing changes in a colon status of mice after 15% HAMSIAA-0.49 treatment provided in Example 6 of the present application;

FIG. 16 is a graph showing changes in colon tissue sections of mice after 15% HAMSIAA-0.49 treatment provided in Example 6 of the present application;

FIG. 17 is a graph showing the content of anti-inflammatory cytokine IL-10 in mouse colon tissue provided in Example 6 of the present application;

FIG. 18 is a graph showing the content of pro-inflammatory cytokine IL-6 in mouse colon tissue provided in Example 6 of the present application;

FIG. 19 is a graph showing the content of pro-inflammatory cytokine IL-1$\beta$ in mouse colon tissue provided in Example 6 of the present application;

FIG. 20 is a graph showing the relative expression level of AhR in mouse colon tissue provided in Example 6 of the present application;

FIG. 21 is a graph showing the content of anti-inflammatory cytokine IL-22 in mouse colon tissue provided in Example 6 of the present application;

FIG. 22 is a comparative picture of HE staining of kidney tissues of mice in each group provided in Example 7 of the present application; and

FIG. 23 shows renal histopathological scores for mice in each group provided in Example 7 of the present application.

## Detailed Description of the Embodiments

The technical solutions of the present application will be clearly and completely described below in conjunction with the examples, and obviously, the described examples are a part of the examples of the present application, rather than all of the examples. Based on the examples in the present application, all other examples obtained by those of ordinary skill in the art without inventive steps fall within the scope of protection of the present application.

According to one aspect of the present application, provided is a starch-indole-acid derivative, prepared mainly by an esterification reaction between amylose and indole acid under the action of a condensing agent and a base; and preferably, the starch-indole-acid derivative after condensation has an indole acid substitution degree of 0.01-1.0.

According to the starch-indole-acid derivative provided by the present application, the starch-indole-acid derivative is mainly prepared by the esterification reaction between the starch and the indole acid under the action of the condensing agent and the base; the starch-indole-acid derivative is acylated starch formed by the esterification reaction between the starch and the indole acid, has high resistance and can resist degradation in the stomach and small intestine. After reaching the colonic site, the starch-indole-acid derivative can be fermented by intestinal flora to release indole acid. Compared with traditional administration methods such as gavage and intraperitoneal injection of indole acid, the described means has significant advantages and can significantly increase the content of indole acid in colon and hepatic portal blood.

It should be noted that inflammatory diseases and autoimmune diseases can be effectively prevented and treated by targeted release of indole acid to a colonic site, wherein the inflammatory diseases and the autoimmune diseases include, but are not limited to, inflammatory bowel disease, type 1 diabetes, systemic lupus erythematosus, rheumatoid arthritis, autoimmune liver disease, multiple sclerosis, and the like.

Preferably, the starch-indole-acid derivative includes indole acid derivatized starch that is one or a mixture of more of indole acetylated starch, indole propionylated starch, indole lactylated starch, and indole acrylated starch.

In a preferred embodiment of the present application, the indole acid includes at least one selected from indoleacetic acid, indolepropionic acid, indoleacrylic acid or indolelactic acid.

In a preferred embodiment of the present application, the starch includes at least one selected from high-amylose maize starch (HAMS), potato starch, sweet potato starch, mixed bean starch and banana starch.

It should be noted that the high-amylose starch refers to starch having an amylose content of more than 50%.

In a preferred embodiment of the present application, the condensing agent includes at least one selected from EDCI, DCC and HATU.

In a preferred embodiment of the present application, the base includes at least one selected from 1-methylimidazole, amines, sodium bicarbonate, and sodium carbonate.

According to one aspect of the present application, provided is a preparation method for the starch-indole-acid derivative, including the steps of:

(a) dissolving starch in a solvent to obtain a solution A; subsequently adding indole acid, a condensing agent and a base into the solution A, and carrying out an esterification reaction to obtain a reaction solution A; and
(b) precipitating the starch in the reaction solution A, followed by successively performing suction filtration and drying to obtain the starch-indole-acid derivative.

According to the preparation method for the starch-indole-acid derivative provided by the present application, the preparation method includes first dissolving the starch in the solvent to obtain the solution A; subsequently adding the indole acid, the condensing agent and the base into the solution A, and carrying out the esterification reaction to obtain the reaction solution A; and then precipitating the starch in the reaction solution A, followed by successively performing suction filtration and drying to obtain the starch-indole-acid derivative. The above preparation method has the advantages of simple processing technology and suitability for industrial large-scale production.

In a preferred embodiment of the present application, the solvent in the step (a) includes at least one selected from DMSO, ionic liquid, and water.

In a preferred embodiment of the present application, the esterification reaction in the step (a) is carried out at a temperature of 20-80°C for 20-30 h.

In a preferred embodiment of the present application, the precipitating in the step (b) is carried out by adding dropwise the reaction solution A into ethanol or water to precipitate the starch-indole-acid derivative.

According to one aspect of the present application, provided is use of the starch-indole-acid derivative in the preparation of a product for regulating intestinal immune balance.

The above starch-indole-acid derivative provided by the present application can be widely used in the preparation of the product for regulating the intestinal immune balance.

In a preferred embodiment of the present application, regulating the intestinal immune balance means that the starch-indole-acid derivative is used as a targeting vector to deliver indole acid to a colon.

Preferably, the product for regulating the intestinal immune balance includes a drug, a health food, a formula food for special medical use or a common food.

The technical solutions of the present application will be further illustrated below in conjunction with the examples.

**Example 1**

(I) Preparation of a starch-indole-acid derivative:

(1) Preparation of indoleacetic acid derivatized high-amylose maize starch (HAMSIAA)

HAMS was added into DMSO, stirring was performed until the obtained solution was clear, IAA, EDCI and 1-methylimidazole were added sequentially, and stirring was continued to be performed for 24 h after material addition was completed. After completion of the reaction, the resulting reaction solution was added dropwise into EtOH or $H_2O$, a solid was precipitated, suction filtration was performed, a filter cake was washed with EtOH or $H_2O$, and drying was performed.

(2) Preparation of indolepropionic acid derivatized high-amylose maize starch (HAMSIPA)

HAMS was added into DMSO, stirring was performed until the obtained solution was clear, IPA, EDCI and 1-methylimidazole were added sequentially, and stirring was continued to be performed for 24 h after material addition was completed. After completion of the reaction, the resulting reaction solution was added dropwise into EtOH or $H_2O$, a solid was precipitated, suction filtration was performed, a filter cake was washed with EtOH or $H_2O$, and drying was performed.

By adjusting a feeding ratio, HAMSIAA and HAMSIPA with different degrees of substitution (0.01-1.0) were obtained. Taking HAMSIAA as an example (Table 1):

Table 1 Preparation of HAMSIAA with different degrees of substitution:

| Group | HAMS feeding amount/g | Reaction condition | Degree of substitution/DS |
|---|---|---|---|
| 1 | 300 | IAA (0.1 eq), EDCI (0.12 eq), N-MIM (0.25 eq), DMSO | 0.06 |
| 2 | 300 | IAA (0.2 eq), EDCI (0.25 eq), N-MIM (0.6 eq), DMSO | 0.12 |
| 3 | 300 | IAA (0.33 eq), EDCI (0.4 eq), N-MIM (0.8 eq), DMSO | 0.19 |
| 4 | 300 | IAA (0.4 eq), EDCI (0.48 eq), N-MIM (1.0eq), DMSO | 0.29 |
| 5 | 300 | IAA (0.6 eq), EDCI (0.7 eq), N-MIM (1.5 eq), DMSO | 0.37 |
| 6 | 300 | IAA (1.0 eq), EDCI (1.2 eq), N-MIM (2.5 eq), DMSO | 0.49 |

(3) Determination of degree of substitution of indole acid derivatized high-amylose maize starch:

The determination of the degree of substitution of indole acid derivatized high-amylose maize starch by titration included two steps of alkaline hydrolysis of ester bonds and neutralization of excess alkaline, and the specific operation was as follows:
0.2 g of starch was weighed, 10 mL of acetone and 1 mL of water were added, sealing was performed, and magnetic stirring was performed until mixing was uniform; 3 mL of an aqueous NaOH solution (1 mol/L) was added, and magnetic stirring was performed for 30 min; 10 mL of hot water of 60°C was added to rinse a bottle wall, and stirring was continued to be performed for 2 min; and the stirred material was allowed to be naturally cooled, 3 drops of a phenolphthalein reagent was added, the solution was titrated with HCl (0.5 mol/L) until the solution became colorless, the solution was allowed to stand at room temperature for 2 min until no color change occurred, and the volume of HCl was recorded. Parallel determinations were performed for 3 times.

A mass fraction $\omega$ of indoleacetyl in the indole acid derivatized high-amylose maize starch HAMSIAA was as follows:

$$\omega = \frac{(V_2 - V_1) \times 10^{-3} \times c \times 158}{m} \times 100\% \tag{1}$$

where V2 and V1 are the volumes of HCl (mL) used for HAMS and HAMSIAA titration, respectively, c is the concentration of HCl (mol/L), m is a mass of an HAMSIAA sample (g), and 158 is a molecular weight of indoleacetyl.

The degree of substitution DS was calculated with the indoleacetyl content as follows:

$$\omega = \frac{158 \times DS}{162 + (158 - 1) \times DS} \times 100\% \tag{2}$$

$$DS = \frac{162 \times \omega}{15800 - 157 \times \omega} \tag{3}$$

wherein 158 is a relative molecular mass of indoleacetyl, 162 is a relative molecular mass per glucose unit of starch, and 1 is a relative atomic mass of an H atom.

By analogy, the degree of substitution of HAMSIPA was as follows:

$$\omega = \frac{(V_2 - V_1) \times 10^{-3} \times c \times 172}{m} \times 100\% \tag{4}$$

$$DS = \frac{162 \times \omega}{17200 - 171 \times \omega} \tag{5}$$

wherein ω is a mass fraction of indolepropionyl in modified starch HAMSIPA, V2 and V1 are the volumes of HCl (mL) used for HAMS and HAMSIPA titration, respectively, c is the concentration of HCl (mol/L), m is a mass of an HAMSIPA sample (g), 172 is a relative molecular mass of indolepropionyl, and 162 is a relative molecular mass of per glucose unit of starch.

(4) Structural characterization of indole acid derivatized starch:

Its specific structural information was characterized by a nuclear magnetic resonance hydrogen spectrum ($^1$H NMR) and an infrared spectrum (FTIR), a crystallinity of modified starch was determined by X-ray diffraction (XRD), and an ultra-micro morphology of starch granules was analyzed by scanning electron microscopy (SEM).

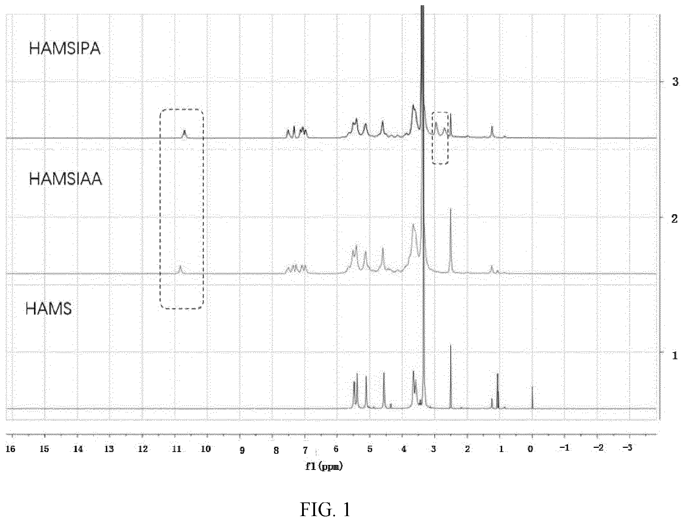

FIG. 1 is a nuclear magnetic resonance hydrogen spectrum of the starch-indole-acid derivative provided in this example; as can be seen from FIG. 1, HAMSIAA and HAMSIPA show a characteristic peak of NH at a chemical shift of 10.83 ppm compared with HAMS, and HAMSIPA shows two new peaks at 2.96 ppm and 2.69 ppm, which are characteristic peaks of two methylene in IPA.

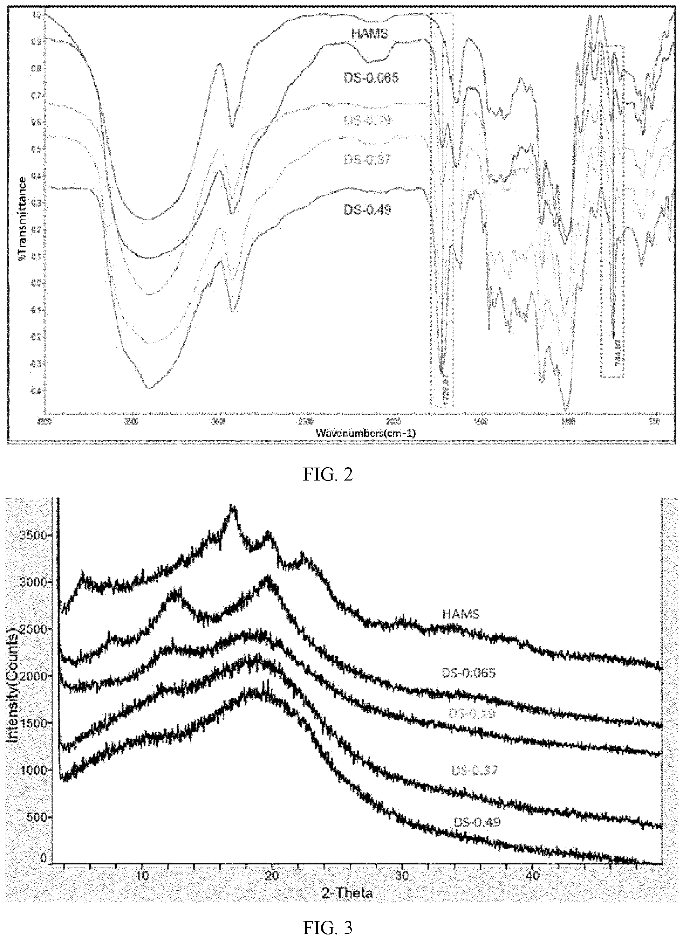

FIG. 2 is an infrared spectrum of the starch (HAMS) and the indoleacetic acid derivatized high-amylose maize starch (HAMSIAA) provided in this example; as can be seen from FIG. 2, a peak at 400 cm$^{-1}$ is an OH or NH stretching vibration peak, the OH peak is broader and the NH peak is sharper, a sharp peak becomes more and more obvious as the degree of substitution increases due to the presence of NH in IAA, and a peak at 2930 cm$^{-1}$ is a CH stretching vibration peak. Compared with HAMS, HAMSIAA shows a new peak at 1728 cm$^{-1}$, which is a stretching vibration peak of carbonyl, and the carbonyl peak at 1728 cm$^{-1}$ becomes stronger as the degree of substitution increases. As can be seen from the spectrum, a peak at 745 cm$^{-1}$ also becomes stronger as the degree of substitution of HAMSIAA increases, which is an out-of-plane bending vibration peak of CH on an aromatic ring, i.e., an out-of-plane bending vibration peak of CH on an indole ring of IAA. It was indicated that an esterification reaction between HAMS and IAA was successful due to the appearance of the carbonyl characteristic peak at 1728 cm$^{-1}$ and the characteristic peak of CH on the aromatic ring at 745 cm$^{-1}$.

Note: DS in FIG. 2 is the degree of substitution.

It should be noted that according to literature reports, starch granules are of a polycrystalline system, and a crystal structure of the starch granules varies with plant varieties from different sources, mainly producing three types of X-ray diffraction patterns (A-type, B-type and C-type). In addition, a V-type structure can be obtained by special methods such as starch acylation, and also certain genetically cultivated starches exhibit A+V, B+V and C+V types. Starch with different crystal forms has obvious characteristic peaks, and the A type has strong peaks at 15°, 17°, 18° and 23°; the B type has strong peaks at 5.6°, 17°, 22° and 24°; the C type shows a combination of the A type and the B type, with a peak appearing at 5.6° compared with the A type, and a strong peak appearing at 23° compared with the B type; and the V type has characteristic peaks at 12.5° and 19.5°.

FIG. 3 is an X-ray diffraction (XRD) pattern of the starch (HAMS) and the indoleacetic acid derivatized high-amylose maize starch (HAMSIAA) provided in this example; as can be seen from FIG. 3, HAMS has peaks at 5.6°, 17°, 19.5° and 22° and the crystal structure is B-type; whereas HAMSIAA has characteristic peaks at 12.5° and 19.5° and the crystal structure is V-type, and the peaks are weaker and weaker as the degree of substitution increases. These results indicated that acylation of HAMS disrupted its crystal structure.

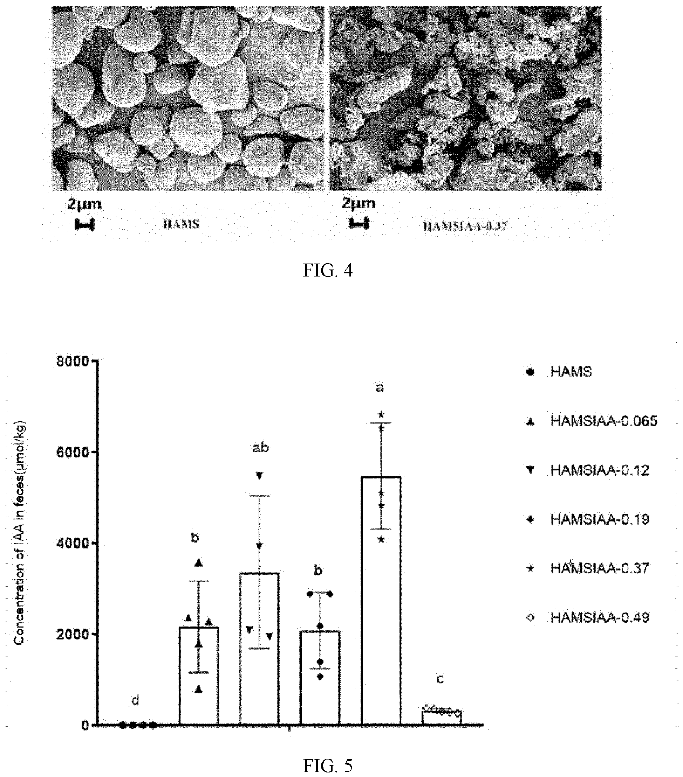

FIG. 4 is a scanning electron microscope (SEM) image of the starch (HAMS) and the indoleacetic acid derivatized high-amylose maize starch (HAMSIAA) provided in this example. As can be seen from FIG. 4, HAMS starch granules are mostly round or oval in shape and have a smooth and non-cracked surface, whereas HAMSIAA starch granules after acylation with IAA have a rough and irregular surface because during the reaction process, dissolving with DMSO was performed, and the resulting reaction solution was added into ethanol or water after the reaction was completed to precipitate the obtained product, which destroyed the granular morphology of the starch.

**Example 2**

Evaluation of the effect of colonic targeted delivery of IAA by indoleacetic acid derivatized starch (HAMSIAA): HAMS and HAMSIAA with different degrees of substitution were used as a mouse feed at an addition rate of 15%, and 8-week-old mice were randomly divided into 6 groups according to the body weight, with 5 mice in each group: an HAMS group and HAMSIAA groups (in the 6 groups, DS was 0.065, 0.12, 0.19, 0.29, 0.37, and 0.49, respectively), and mouse feces and serum were collected.

(1) Method for determination of the content of IAA/IPA in feces, hepatic portal blood, peripheral blood and colon tissues of mice;

A high performance liquid chromatography-tandem mass spectrometry (HPLC-MS/MS) technique was used to detect the content of metabolites IAA/IPA in each sample separately. Determination of the content of IAA/IPA in mouse feces: methanol was added into the collected feces, then vortexing, ultrasound extraction, and centrifuging were performed, a supernatant was taken to obtain an extract, the obtained extract was treated, IAA/IPA internal standards (IAA-d5/IPA-d2) were added for calibration, then purification was performed by using a small HLB solid-phase extraction column (pretreated with 5 mL of methanol and 5 mL water, then rinsed with 5 mL of a methanol/water mixed solution and 5 mL of water, an effluent being discarded, and finally eluted with 5 mL of methanol before use), and an eluate was analyzed by HPLC-MS/MS to detect IAA/IPA. Determination of the content of IAA/IPA in the hepatic portal blood and the peripheral blood: whole blood collected was allowed to stand and centrifuged at low temperature, and a supernatant was taken to obtain serum, and then an internal standard was added into the serum, purification was performed by using a solid-phase extraction column, and metabolites IAA/IPA in an eluate were detected by HPLC-MS/MS by the same method as described above. Determination of the content of IAA/IPA in the colon tissues: methanol was added into colon tissues collected, ultrasonically disrupted and further uniformly ground, then vortexing, ultrasound extraction, and centrifuging were performed, a supernatant was taken to obtain an extract, and then an internal standard was added into serum, purification was performed by using a solid-phase extraction column, and the concentration of IAA/IPA in the extract was detected by HPLC-MS/MS by the same method as described above.

FIG. 5 is a graph showing the concentration of indole acid in feces of experimental mice in this example. As can be seen from FIG. 5, when modified starch was added in an amount of 15% into a feed, the concentration of IAA in the feces could be significantly increased in the HAMSIAA groups compared with the HAMS group. Wherein when the degree of substitution of HAMSIAA was 0.37, the concentration of IAA in the feces was the highest, about 5470 $\mu$mol/kg, which was improved by about 1370 times compared with the HAMS group (about 4 $\mu$mol/kg). This indicates that the carrying capacity of IAA is related to the degree of substitution, and when the degree of substitution is lower, the concentration of IAA in the feces increases as the degree of substitution increases, but decreases when the degree of substitution reaches a certain value and then increases again.

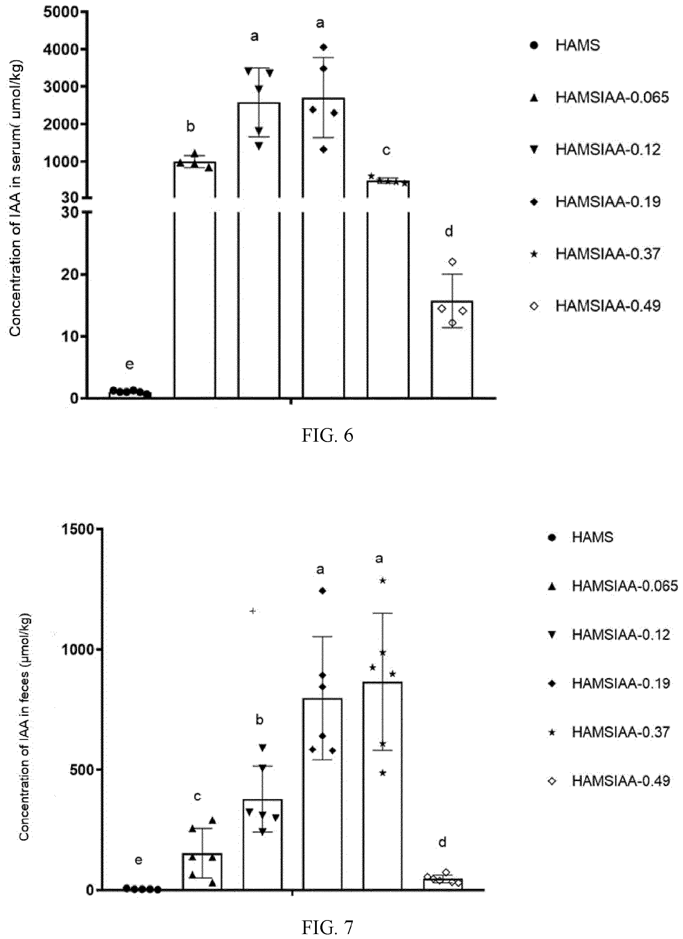

FIG. 6 is a graph showing the concentration of indole acid in hepatic portal blood of experimental mice in this example. As can be seen from FIG. 6, when the modified starch was added in the amount of 15% into the feed, the concentration of IAA in the hepatic portal blood could be significantly increased in the HAMSIAA groups compared with the HAMS group. A law of variation of the concentration of IAA in the hepatic portal blood was slightly different from that in feces in the groups of HAMSIAA with different degrees of substitution, wherein the concentration of IAA in the hepatic portal blood was the highest in the group of HAMSIAA with a degree of substitution of 0.19, and was about 2700 nmol/mL, which was improved by about 2700 times compared with the HAMS group (about 1 nmol/mL); while the concentration of IAA in the feces was the highest in the group of HAMSIAA with a degree of substitution of 0.37, probably because HAMSIAA with the degree of substitution of 0.37 has high resistance, is less absorbed in the stomach or small intestine, and is more decomposed and absorbed after reaching a colonic site.

By detecting the concentration of IAA in the feces and the hepatic portal blood of the mice, we found that when the modified starch was added in the amount of 15% into the feed, the concentration of IAA was extremely significantly increased in the HAMSIAA groups compared with the HAMS group, indicating that HAMSIAA could well achieve targeted delivery of IAA, and made IAA slowly released to the colonic site, wherein HAMSIAA with a degree of substitution of 0.37 had the best targeted delivery effect.

## Example 3

Evaluation of the effect of colonic targeted delivery of IAA by indoleacetic acid derivatized starch (HAMSIAA): HAMS and HAMSIAA with different degrees of substitution were used as a mouse feed at an addition rate of 1.5%, and 8-week-old mice were randomly divided into 6 groups according to the body weight, with 5 mice in each group: an HAMS group and HAMSIAA groups (in the 6 groups, DS was 0.065, 0.12, 0.19, 0.29, 0.37, and 0.49, respectively), and mouse feces and serum were collected.

FIG. 7 is a graph showing the concentration of indole acid in feces of experimental mice in this example. As can be seen from FIG. 7, when the amount of the modified starch added into the feed was reduced to 1.5%, the concentration of IAA in the feces could still be significantly increased in the HAMSIAA groups compared with the HAMS group, and the concentration of IAA in the feces was the highest in the group of HAMSIAA with a degree of substitution of 0.37, and was about 860 $\mu$mol/kg, which was improved by about 200 times compared with the HAMS group (about 4 $\mu$mol/kg).

## Example 4

Evaluation of the effect of targeted delivery of IAA by different modes of administration:
The experiments were divided into 5 groups in total:

1. Blank control group;
2. HAMSIAA(1.5%) - 0.32 group (with an average daily consumption of 3g of mouse food per mouse, and a total intake of 0.068 mmol IAA);
3. IAANa-containing water drinking group (with the IAANa concentration of 2.68 mg/mL, average daily drinking of 5 mL of water per mouse, and a total intake of 0.068 mmol IAANa);
4. Gavage group (with the IAANa concentration of 67 mg/mL, daily gavage of 0.2 mL, and a total intake of 0.068 mmol IAANa); and
5. Intraperitoneal injection group (with the IAANa concentration of 67 mg/mL, daily injection of 0.2mL, and a total intake of 0.068 mmol IAANa).

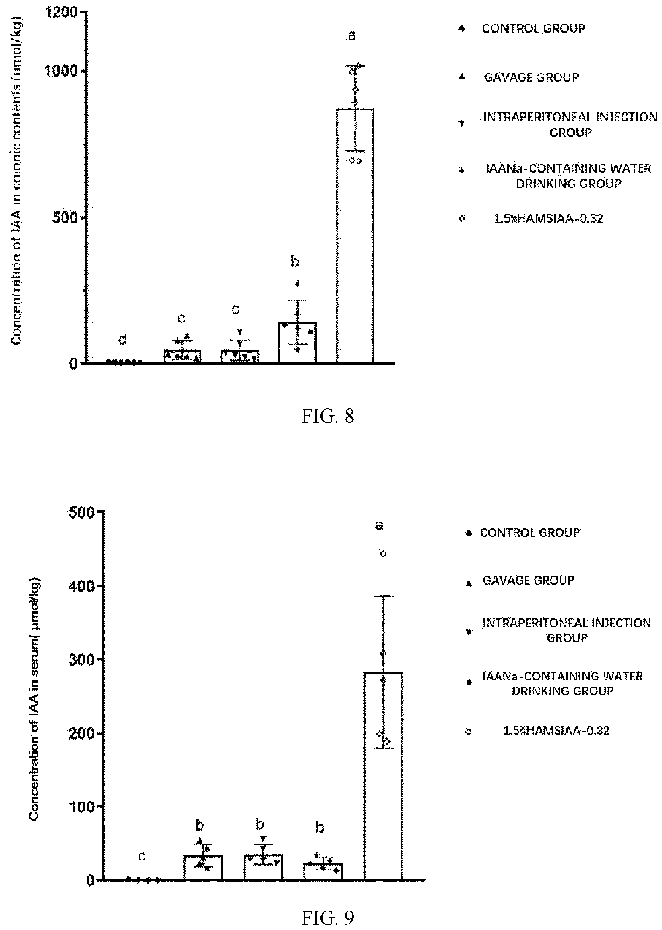

The effect of colonic targeted delivery of IAA by different modes of administration was evaluated by comparing the concentration of IAA in colonic contents and hepatic portal blood of mice after four modes of administration, i.e., gavage, intraperitoneal injection, direct drinking of saline containing IAA, and HAMSIAA, and the results are shown in FIG. 8:
FIG. 8 shows the concentration of IAA in colonic contents for different modes of administration.

As can be seen from FIG. 8, HAMSIAA can more efficiently deliver IAA to a colonic site compared with the three modes of administration, i.e., gavage, intraperitoneal injection, and direct drinking of the saline containing IAA. When IAA intake was comparable, the concentration of IAA in feces was significantly improved in the HAMSIAA group, which was improved by about 200 times compared with the control group; the concentration of IAA in feces in the gavage group and the intraperitoneal injection group was comparable and improved by about 7 times compared with the control group; and the concentration of IAA in feces in the IAANa-containing water drinking group was improved by about 30 times compared with the control group. The experimental results indicated that whether gavage or direct drinking of the saline containing IAA, the majority was absorbed in the stomach or small intestine, and only a small portion reached the colonic site, while HAMSIAA could resist digestion in the stomach and small intestine using resistant starch, reached the colonic site, and slowly released IAA via intestinal microbial fermentation at the colonic site. By detecting the concentration of IPA in the colonic contents of mice after different modes of administration, it was found that the IPA concentration in the gavage group, the intraperitoneal injection group and the IAANa-containing water drinking group was not significantly changed compared with the control group, while the IPA concentration in the HAMSIAA group was significantly increased compared with the control group.

FIG. 9 shows the concentration of IAA in hepatic portal blood serum for different modes of administration; as can be seen from FIG. 9, when IAA intake was comparable, there was a significant increase in the concentration of IAA in the hepatic portal blood of mice after different modes of administration compared with the control group. There were no significant differences in the concentration of IAA in serum in the gavage group, the intraperitoneal injection group, and the IAANa-containing water drinking group, whereas the concentration of IAA in serum was improved by about 10 times in the 1.5% HAMSIAA-0.32 group compared with the other administration modes. This illustrates that the targeted delivery and absorption effect of HAMSIAA is a significant advantage over other modes of administration.

### Example 5

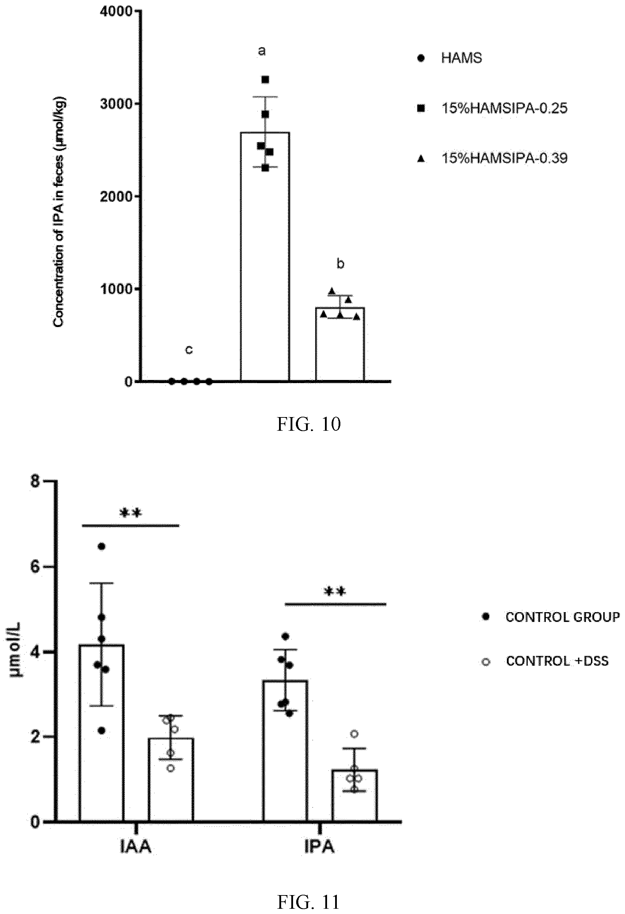

Evaluation of the effect of colonic targeted delivery of IPA by indolepropionic acid derivatized starch (HAMSIPA): Indole acid derivatized starches with different degrees of substitution were added to a mouse feed in a ratio of 15% and 1.5%, respectively, and feces were taken to test the IPA content after mice were fed with a special feed prepared for one week. The results are shown in FIG. 10:

FIG. 10 is a graph showing the concentration of IPA in feces of experimental mice provided in this example.

As can be seen from FIG. 10, the concentration of IPA in the feces was significantly increased in the HAMSIPA group compared with the HAMS group, with the degree of substitution and dose dependence, the more the addition amount, the higher the concentration of IPA in the feces. The concentration of IPA in the feces was higher in the HAMSIPA group with a degree of substitution of 0.25. It is indicated that HAMSIPA also has the good function of colonic targeted delivery of IPA.

### Example 6

HAMSIAA alleviates dextran sulfate sodium (DSS)-induced acute ulcerative colitis: Mouse food was prepared from the prepared HAMSIAA in a certain addition ratio, and mice were divided into a control mouse food group, a HAMS group, a control mouse food+DSS group, a HAMS+DSS group, and HAMSIAA with different degrees of substitution+DSS groups. Fecal morphological changes, a mental state and a body weight of the mice were observed and recorded daily after the start of the experiment, ordinary drinking water and an aqueous DSS solution were changed once every 3 days, and the aqueous DSS solution was changed to the ordinary drinking water on day 7 after the start of the experiment.

After the end of the experiment, a disease active index (DAI) of mice was evaluated, mouse colons were taken to measure their lengths, and a colon status of the mice was evaluated by sectioning, and the levels of cytokines IL-10, IL-6, IL-22, etc. in colon tissues of the mice were detected. The effect of HAMSIAA on the prevention and treatment of DSS-induced acute ulcerative colitis in mice was finally evaluated by these indicators.

FIG. 11 is a comparative diagram of IAA/IPA concentrations in peripheral blood serum in a normal group and a dextran sulfate sodium (DSS)-induced ulcerative colitis group;

As can be seen from FIG. 11, the concentration of IAA/IPA in serum in the control+DSS group was significantly reduced compared with the control group, indicating that intestinal microbial metabolites IAA/IPA of tryptophan are related to ulcerative colitis. Therefore, we speculated that the severity of DSS-induced ulcerative colitis in mice would be alleviated by supplementing with IAA/IPA.

Further, in the present application, by taking 15% HAMSIAA-0.49 as an example, it was demonstrated that the addition of HAMSIAA in a feed could effectively relieve the severity of DSS-induced ulcerative colitis in mice by indicators such as the body weight change, the colon length, the DAI, the colon status and colon tissue sections, wherein:

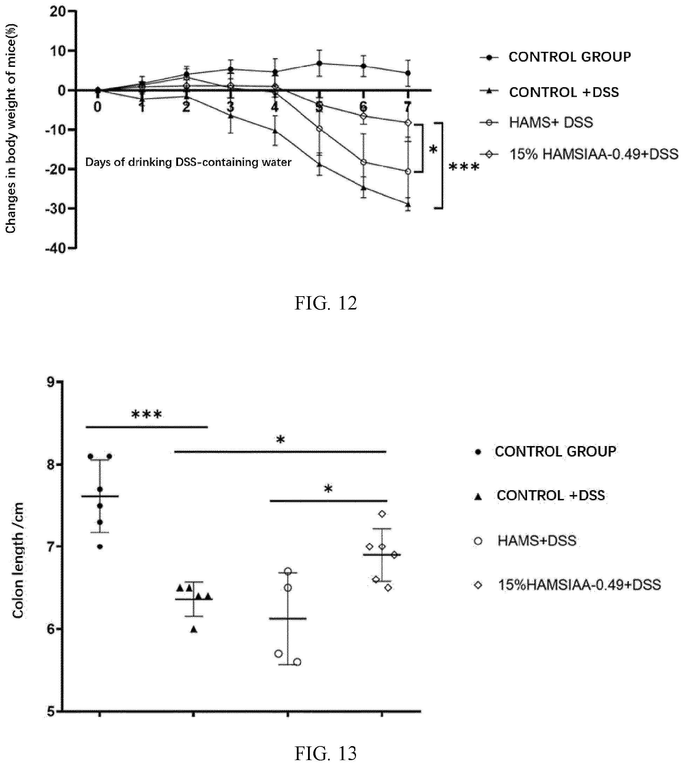

FIG. 12 is a graph showing changes in a body weight of mice after 15% HAMSIAA-0.49 treatment;
FIG. 13 is a graph showing changes in a colon length of mice after 15% HAMSIAA-0.49 treatment;

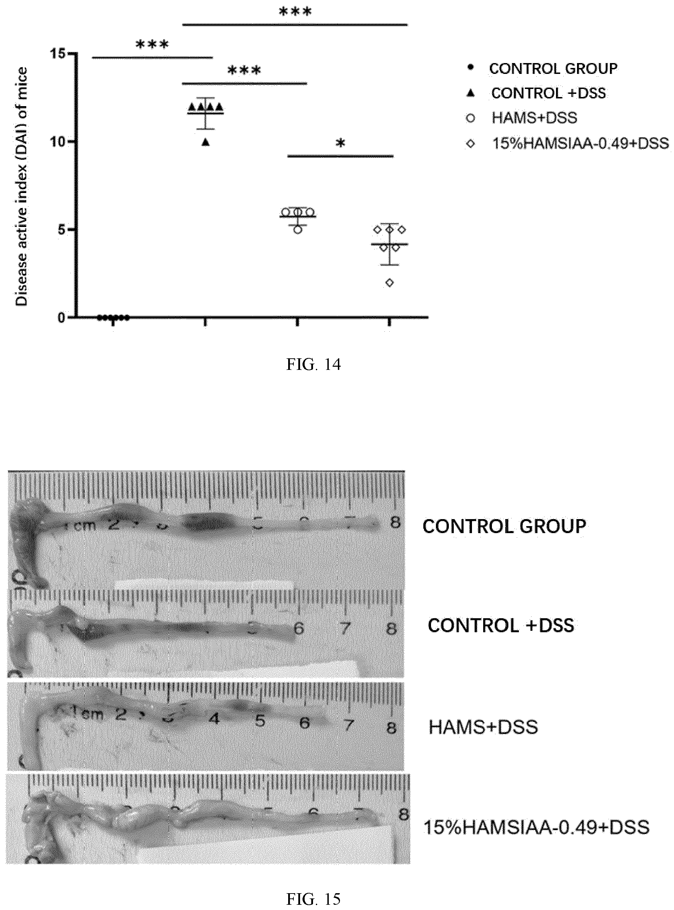

FIG. 14 is a graph showing changes in a disease active index (DAI) of mice after 15% HAMSIAA-0.49 treatment;
FIG. 15 is a graph showing changes in a colon status of mice after 15% HAMSIAA-0.49 treatment;

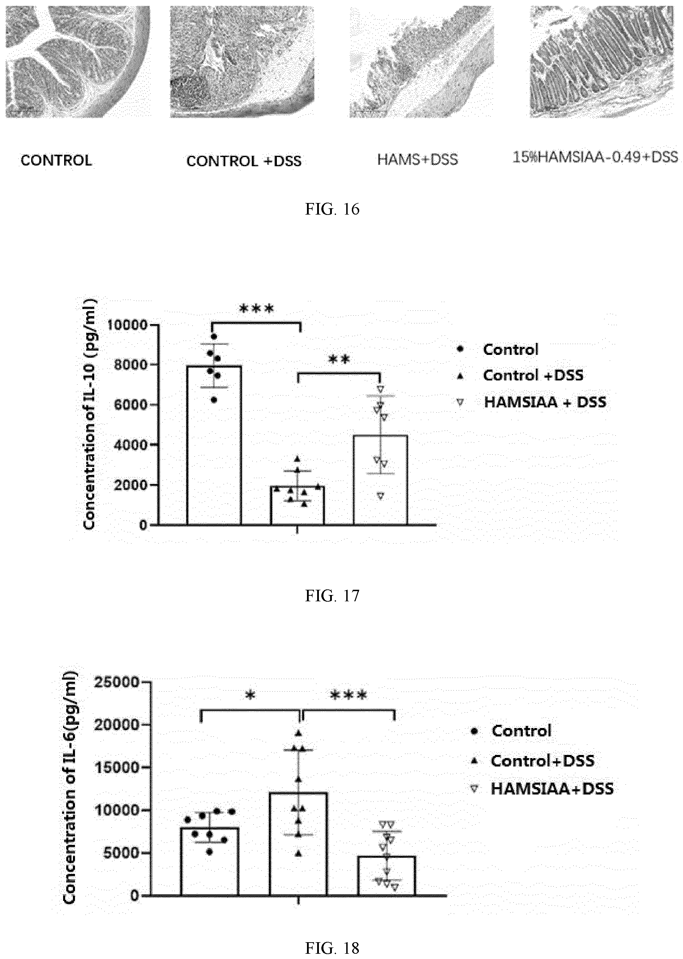

FIG. 16 is a graph showing changes in colon tissue sections of mice after 15% HAMSIAA-0.49 treatment;
As can be seen from FIG. 12 to FIG. 16, compared with the control group, the body weight of mice in the model group (control+DSS) was significantly decreased, the weight loss in the HAMS+DSS group was relieved, and the weight loss in the 15% HAMSIAA-0.49+DSS group was significantly slowed, which was significantly different from those in both the model group and the HAMS+DSS group (FIG. 12); compared with the control group, the colon length in the model group was significantly shortened, there was no significant difference between the HAMS group and the model group, while the colon length in the 15% HAMSIAA-0.49+DSS group was longer compared with the model group, and there was a significant difference (FIG. 13); compared with the model group, the DAI in the 15% HAMSIAA-0.49+DSS group was also decreased significantly, and there was a significant difference (FIG. 14); and FIG. 15 shows pictures of colons in the control group, the control+DSS group, the HAMS+DSS group, and the 15% HAMSIAA-0.49+DSS group, and the colon status was the best in the 15% HAMSIAA-0.49+DSS group. Compared with the control group, the colon tissue in the model group was severely damaged, with lesions such as glandular disappearance and extensive necrosis of a mucosal layer, and compared with the model group, the degree of colon damage in the HAMS+DSS group was slightly reduced, with a small number of intestinal glands of a normal structure, while the protective effect of HAMSIAA-0.49 on the colon tissues in mice was most pronounced (FIG. 16). The body weight change, the colon length, the DAI, and the tissue sections all demonstrated that HAMSIAA could effectively alleviate the severity of DSS-induced ulcerative colitis in mice.

Further, in the present application, by taking 1.5% HAMSIAA-0.32 as an example, it was further demonstrated that the addition of HAMSIAA in a feed could effectively alleviate the severity of DSS-induced ulcerative colitis in mice by detecting the levels of cytokines IL-10, IL-6, IL-1β, IL-22, etc. and the relative expression levels of mRNA (AhR/GAPDH) in mouse colon tissue, and that the alleviation of DSS-induced ulcerative colitis in mice by HAMSIAA may be closely related to the activation of an AHR/IL-22 pathway, wherein:

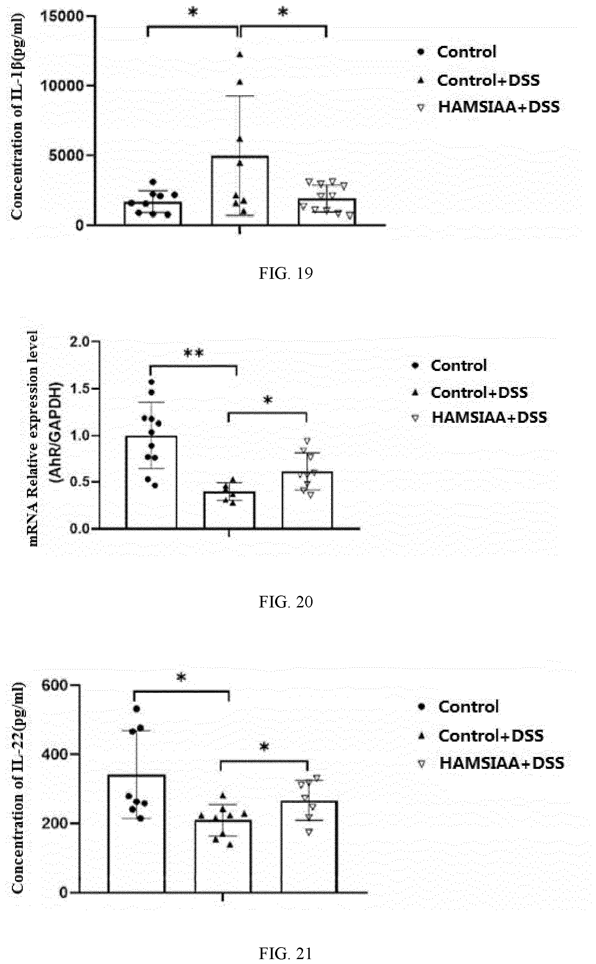

FIGS. 17 to 19 demonstrate from cytokine levels that HAMSIAA effectively alleviates the severity of DSS-induced ulcerative colitis in mice. IL-10 is an important anti-inflammatory cytokine secreted by immune and non-immune cells. The occurrence of colitis is related to a decrease in IL-10, the expression of IL-10 is also related to disease progression, and with the aggravation of the lesion extent, the expression of II,-10 shows a downward trend, but begins to increase in a remission period, indicating that IL-10 begins to exert its inflammatory inhibitory effect to restore the balance between inflammatory factors and anti-inflammatory factors. Compared with the model group, the level of IL-10 was significantly increased in the colon tissue in the HAMSIAA treatment group, indicating that HAMSIAA effectively alleviated DSS-induced colitis (FIG. 17). IL-6 and IL-1β are important pro-inflammatory cytokines. The levels of IL-6 and IL-1β are significantly elevated in patients with colitis. Compared with the model group, the levels of IL-6 and IL-1β in the colon tissue were significantly reduced in the HAMSIAA treatment group, further indicating that HAMSIAA effectively alleviated DSS-induced colitis (FIG. 18 and FIG. 19).

FIGS. 20 and 21 indicate that the improvement in HAMSIAA treatment of colitis may be closely related to activation of the AHR/IL-22 pathway. By detecting the relative expression level of mRNA (AhR/GAPDH) in the colon tissue, it was found that the relative expression of AhR was significantly increased in the HAMSIAA treatment group compared with the model group, indicating that IAA binds to AhR, promoting the expression of AhR (FIG. 20). By detecting the level of IL-22 in the colon tissue, it was found that the content of IL-22 was significantly increased in the HAMSIAA treatment group compared with the model group (FIG. 21). IL-22 has the ability to constitutively activate STAT3, which promotes epithelial cell regeneration and enhances the integrity of a mucosal barrier by stimulating the expression of antimicrobial peptides and mucins. IL-22 from group 3 innate lymphoid cells is beneficial to an intestinal barrier, while the expression of IL-22 is controlled by AhR activation. This is strong evidence of IAA binding to AhR. Thus, the improvement in HAMSIAA treatment of colitis may be closely related to activation of the AHR/IL-22 pathway.

**Example 7**

HAMSIAA alleviates systemic lupus erythematosus (SLE):

1. Mouse food was prepared from the prepared HAMSIAA in a certain addition ratio, and SLE model mice and control mice were purchased from Changzhou Cavens Experimental Animal Co., Ltd, aged 4-6 weeks. All mice were randomly divided into 3 groups:

Control group (MRL/MPJ);
Systemic lupus erythematosus model group (MRL/lpr); and
HAMSIAA group (MRI,/lpr+HAMSIAA).

The HAMSIAA group was given a special feed in which HAMSIAA was added in a certain proportion for 14 weeks. Kidney tissues were collected after the end of the experiment.
2. In the present application, by taking 1.5% HAMSIAA-0.32 as an example, it was demonstrated that the addition of HAMSIAA in the feed could effectively relieve SLE by a renal pathological index, wherein:

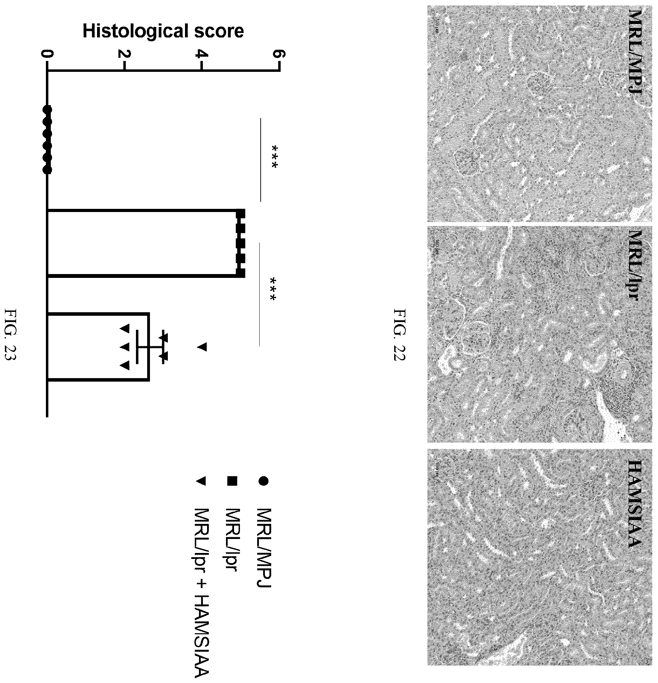

FIG. 22 is a comparative picture of HE staining of kidney tissues of mice in each group in this example;
FIG. 23 shows renal histopathological scores for mice in each group in this example.

Both HE staining of the kidney tissues of the mice and renal histopathological scores showed that the use of HAMSIAA could effectively alleviate glomerulonephritis and interstitial nephritis in mice compared with the SLE mouse model group. These results indicated that the use of HAMSIAA could effectively alleviate symptoms in SLE mice.

Finally, it should be described that the above examples are only used to illustrate the technical solutions of the present application, but not to limit the technical solutions of the present application; although the present application has been described in detail with reference to the foregoing examples, it should be understood by those of ordinary skill in the art that modifications may still be made to the technical solutions described in the foregoing examples or equivalents substitutions may still be made to some or all of the technical features in the technical solutions; and these modifications or substitutions do not make the essence of the corresponding technical solutions depart from the scope of the technical solutions of the examples of the present application.

Industrial applicability: the starch-indole-acid derivative provided by the present application is acylated starch formed by the esterification reaction between the starch and the indole acid, has high resistance and can resist degradation in the stomach and small intestine. After reaching the colonic site, the starch-indole-acid derivative can be fermented by intestinal flora to release indole acid. Compared with traditional administration methods such as gavage and intraperitoneal injection of indole acid, the described means has significant advantages and can significantly increase the content of indole acid in colon and hepatic portal blood, and thus, the starch-indole-acid derivative of the present application can be widely used in the product for regulating the intestinal immune balance, and the product for preventing and treating inflammatory diseases. Meanwhile, the preparation method for the starch-indole-acid derivative of the present application includes first dissolving the starch in the solvent to obtain the solution A; subsequently adding the indole acid, the condensing agent and the base into the solution A, and carrying out the esterification reaction to obtain the reaction solution A; and then precipitating the starch in the reaction solution A, followed by successively performing suction filtration and drying to obtain the starch-indole-acid derivative. The above preparation method has the advantages of simple processing technology and suitability for industrial large-scale production.

Claims

1.    A starch-indole-acid derivative, **characterized by** being mainly prepared by an esterification reaction between starch and indole acid under the action of a condensing agent and a base, wherein
the starch-indole-acid derivative after condensation has an indole acid substitution degree of 0.01-1.0.

2.    The starch-indole-acid derivative according to claim 1, **characterized in that** the indole acid comprises at least one selected from indoleacetic acid, indolepropionic acid, indoleacrylic acid or indolelactic acid.

3.    The starch-indole-acid derivative according to claim 1, **characterized in that** the starch comprises at least one selected from high-amylose maize starch, potato starch, sweet potato starch, mixed bean starch and banana starch.

4.    The starch-indole-acid derivative according to claim 1, **characterized in that** the condensing agent comprises at least one selected from EDCI, DCC and HATU.

5.    The starch-indole-acid derivative according to claim 1, **characterized in that** the base comprises at least one selected from 1-methylimidazole, amines, sodium bicarbonate, and sodium carbonate.

6.    The starch-indole-acid derivative according to claim 1, **characterized by** comprising any one selected from indoleacetic acid derivatized high-amylose maize starch and indolepropionic acid derivatized high-amylose maize starch.

7.    The starch-indole-acid derivative according to claim 6, **characterized in that** when the starch-indole-acid derivative is the indoleacetic acid derivatized high-amylose maize starch, the indoleacetic acid derivatized high-amylose maize starch has an indole acid substitution degree of 0.065-0.49.

8.    The starch-indole-acid derivative according to claim 6, **characterized in that** when the starch-indole-acid derivative is the indolepropionic acid derivatized high-amylose maize starch, the indolepropionic acid derivatized high-amylose maize starch has an indole acid substitution degree of 0.25-0.39.

9. A preparation method for the starch-indole-acid derivative according to any one of claims 1-8, **characterized by** comprising the steps of:

(a) dissolving starch in a solvent to obtain a solution A; and subsequently adding indole acid, a condensing agent and a base into the solution A, and carrying out an esterification reaction to obtain a reaction solution A; and
(b) precipitating the starch in the reaction solution A, followed by successively performing suction filtration, washing, and drying to obtain the starch-indole-acid derivative.

10. The preparation method for the starch-indole-acid derivative according to claim 9, **characterized in that** the solvent in the step (a) comprises at least one selected from DMSO, ionic liquid, and water.

11. The preparation method for the starch-indole-acid derivative according to claim 9, **characterized in that** the esterification reaction in the step (a) is carried out at a temperature of 20-80°C for 20-30 h.

12. The preparation method for the starch-indole-acid derivative according to claim 9, **characterized in that** the precipitating in the step (b) is carried out by adding dropwise the reaction solution A into ethanol or water to precipitate the starch-indole-acid derivative.

13. Use of the starch-indole-acid derivative according to any one of claims 1-8 in the preparation of a product for regulating intestinal immune balance.

14. The use according to claim 13, **characterized in that** regulating the intestinal immune balance means that after being ingested and reaching a colonic site, the starch-indole-acid derivative is fermented by intestinal flora to release linked indole acid, which cooperates with short-chain fatty acids released by fermentation of starch by intestinal flora, synergistically exerting immunomodulatory effects by means of multiple immune system signaling pathways.

15. The use according to claim 13, **characterized in that** the product for regulating the intestinal immune balance comprises a drug, a health food, a formula food for special medical use or a common food.

16. The use according to claim 15, **characterized in that** the drug is a drug for the prevention and treatment of inflammatory diseases and/or autoimmune diseases.

17. The use according to claim 15, **characterized in that** the inflammatory diseases and/or the autoimmune diseases comprise one selected from inflammatory bowel disease, type 1 diabetes, systemic lupus erythematosus, rheumatoid arthritis, autoimmune liver disease, and multiple sclerosis.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

CONTROL        CONTROL +DSS        HAMS+DSS       15%HAMSIAA-0.49+DSS

FIG. 16

FIG. 17

FIG. 18

FIG. 19

FIG. 20

FIG. 21

FIG. 22

FIG. 23

## INTERNATIONAL SEARCH REPORT

| International application No. |
| --- |
| **PCT/CN2022/125134** |

**A. CLASSIFICATION OF SUBJECT MATTER**

C08B 31/04(2006.01)i; C08B 33/02(2006.01)i; A23L 33/125(2016.01)i; A61P 29/00(2006.01)i

According to International Patent Classification (IPC) or to both national classification and IPC

**B. FIELDS SEARCHED**

Minimum documentation searched (classification system followed by classification symbols)

C08B; A23L; A61P

Documentation searched other than minimum documentation to the extent that such documents are included in the fields searched

Electronic data base consulted during the international search (name of data base and, where practicable, search terms used)

CNTXT, CNABS, CNKI, 万方, WANFANG, VEN, ENTXT, ISI Web of Knowledge: 肠, 吲哚美辛, 吲哚, 吲哚酸, 乙酸, 丙酸, 丙烯酸, 乳酸, 淀粉, 酯化, 苯并吡咯, 免疫, starch, amylose, indole, indomethacin, acetic, propionic, acrylic, lactic, acid

**C. DOCUMENTS CONSIDERED TO BE RELEVANT**

| Category* | Citation of document, with indication, where appropriate, of the relevant passages | Relevant to claim No. |
| --- | --- | --- |
| X | 彭宁福 (PEN, Ningfu). "吲哚美辛结肠靶向前药的合成以及抑制裸鼠结肠癌肝转移的研究 (Non-official translation: Synthesis of Indomethacin Colon-targeted Prodrug and Research on Inhibition of Hepatic Metastasis of Colonic Carcinoma in Nude Mouse)" 万方数据库 *(WANFANG DATA)*, 25 September 2008 (2008-09-25), page 7, last paragraph to page 8, figures 1-3, and page 11, table 1-1 to page 15, table 1-5 | 1-12 |
| Y | 彭宁福 (PEN, Ningfu). "吲哚美辛结肠靶向前药的合成以及抑制裸鼠结肠癌肝转移的研究 (Non-official translation: Synthesis of Indomethacin Colon-targeted Prodrug and Research on Inhibition of Hepatic Metastasis of Colonic Carcinoma in Nude Mouse)" 万方数据库 *(WANFANG DATA)*, 25 September 2008 (2008-09-25), page 7, last paragraph to page 8, figures 1-3, and page 11, table 1-1 to page 15, table 1-5 | 13-17 |
| Y | 邱月琴等 (QIU, Yueqin et al.). "色氨酸微生物代谢产物的肠道免疫调节作用及其机制 (Modulation Effect of Tryptophan Metabolites from Microbiota on Intestinal Immune and Its Mechanism)" 动物营养学报 *(Chinese Journal of Animal Nutrition)*, Vol. 1, No. 31, 02 November 2018 (2018-11-02), page 43, right column, paragraph 1 | 13-17 |

☑ Further documents are listed in the continuation of Box C.    ☑ See patent family annex.

| | |
| --- | --- |
| *   Special categories of cited documents: <br> "A"   document defining the general state of the art which is not considered to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
| --- | --- |
| **19 December 2022** | **28 December 2022** |

| Name and mailing address of the ISA/CN | Authorized officer |
| --- | --- |
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/125134** |

**C.     DOCUMENTS CONSIDERED TO BE RELEVANT**

| Category* | Citation of document, with indication, where appropriate, of the relevant passages | Relevant to claim No. |
| --- | --- | --- |
| X | CAI, Xiang et al. "Evaluation of Amylose Uesd as a Drug Delivery Carrier" *Carbohydrate Research,* No. 345, 17 February 2010 (2010-02-17), pages 922-928 | 1-12 |
| Y | CAI, Xiang et al. "Evaluation of Amylose Uesd as a Drug Delivery Carrier" *Carbohydrate Research,* No. 345, 17 February 2010 (2010-02-17), pages 922-928 | 13-17 |
| X | 蔡祥 (CAI, Xiang). "基于多糖载体的高分子前药研究 (Non-official translation: Polymeric Prodrug Research Based on Polysaccharide Carrier)" 万方数据库 *(WANFANG DATA),* 21 September 2009 (2009-09-21), pages 76-77 | 1-12 |
| Y | 蔡祥 (CAI, Xiang). "基于多糖载体的高分子前药研究 (Non-official translation: Polymeric Prodrug Research Based on Polysaccharide Carrier)" 万方数据库 *(WANFANG DATA),* 21 September 2009 (2009-09-21), pages 76-77 | 13-17 |
| PX | CN 113912743 A (SHANDONG SHANWEI IMMUNOLOGY TECHNOLOGY CO., LTD.) 11 January 2022 (2022-01-11) claims, and drawings | 1-17 |
| A | CN 109620825 A (PEKING UNIVERSITY PEOPLE'S HOSPITAL (THE SECOND CLINICAL SCHOOL OF MEDICINE, PEKING UNIVERSITY)) 16 April 2019 (2019-04-16) entire document | 1-17 |
| A | CN 109730993 A (PEKING UNIVERSITY PEOPLE'S HOSPITAL (THE SECOND CLINICAL SCHOOL OF MEDICINE, PEKING UNIVERSITY)) 10 May 2019 (2019-05-10) entire document | 1-17 |

Form PCT/ISA/210 (second sheet) (January 2015)

**INTERNATIONAL SEARCH REPORT**
Information on patent family members

| International application No. |
| :-- |
| **PCT/CN2022/125134** |

| Patent document cited in search report | | | Publication date (day/month/year) | Patent family member(s) | | | Publication date (day/month/year) |
| :-- | :-- | :-- | :-- | :-- | :-- | :-- | :-- |
| CN | 113912743 | A | 11 January 2022 | None | | | |
| CN | 109620825 | A | 16 April 2019 | CN | 109620825 | B | 09 February 2021 |
| CN | 109730993 | A | 10 May 2019 | None | | | |

Form PCT/ISA/210 (patent family annex) (January 2015)

27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111213344